# EUROPEAN PATENT APPLICATION

(11) **EP 4 467 003 A1**
(43) Date of publication of application: **27.11.2024**
(21) Application number: 23899649.0
(22) Date of filing: 31.10.2023
(51) Int. Cl.: A23K 10/12, A23K 10/38, A23K 10/14, A23K 30/00

(54) **FERMENTED FEED BASED ON WHOLE SPENT GRAINS AND PREPARATION METHOD THEREFOR**

(30) Priority: 28.12.2022 CN 202211693065
(71) Applicant: Tsingtao Brewery Co., Ltd., Shibei District Qingdao, Shandong 266000 (CN)
(72) Inventor: XU, Nan, Qingdao, Shandong 266000 (CN); YIN, Hua, Qingdao, Shandong 266000 (CN); YU, Junhong, Qingdao, Shandong 266000 (CN); HUANG, Shuxia, Qingdao, Shandong 266000 (CN); YAN, Peng, Qingdao, Shandong 266000 (CN); LIU, Jia, Qingdao, Shandong 266000 (CN); QIN, Qingqing, Qingdao, Shandong 266000 (CN); WANG, Jianfeng, Qingdao, Shandong 266000 (CN); HU, Shumin, Qingdao, Shandong 266000 (CN); WANG, Shuqian, Qingdao, Shandong 266000 (CN); LIU, Mingli, Qingdao, Shandong 266000 (CN)
(74) Representative: J A Kemp LLP
(86) International application number: PCT/CN2023/128347
(87) International publication number: WO 2024/120068

(57) **Abstract**

The present application discloses a fermented feed based on whole spent grains and a preparation method therefor, belonging to the technical field of fermented feed. The method includes a fresh-keeping treatment step, a drying step, an aerobic fermentation step and an anaerobic fermentation step; the fresh-keeping treatment step includes: adding lactic acid bacteria to wet spent grains immediately after taking the wet spent grains; the aerobic fermentation step includes: adding mixed microbial community and enzyme preparations to the spent grains obtained by the drying step, and performing aerobic fermentation, the mixed microbial community includes yeast and *Bacillus,* the enzyme preparations include complex cellulases, proteases and xylanases; the anaerobic fermentation step includes: adding lactic acid bacteria to the spent grains obtained by the aerobic fermentation step, and performing anaerobic fermentation to obtain the fermented feed. The present application is applied to animal feeding feed, and solves the problems of the existing grains processing technology, such as the grains are easy to deteriorate, the nutritional value of the grains cannot be effectively utilized, and the fermentation technology is imperfect.

## Description

The present application claims priority to Chinese Patent Application No. 202211693065.X filed to the CNIPA on December 28, 2022 and entitled "FERMENTED FEED BASED ON WHOLE SPENT GRAINS AND PREPARATION METHOD THEREFOR", the disclosure of which is hereby incorporated by reference in its entirety.

### TECHNICAL FIELD

The present application belongs to the technical field of fermented feed, and in particular, relates to a fermented feed based on whole spent grains and a preparation method therefor.

### BACKGROUND ART

Brewer's grains are the main by-product in the beer brewing industry, accounting for about 8-10% of beer production (wet weight, moisture content of about 80%). China's beer production has ranked first in the world for 20 consecutive years. Taking the 2021 beer production of 35.62 million kiloliters as an example, the output of brewer's grains was about 3 million tons, which is a very large amount. Brewer's grains are the by-product of the saccharification process in beer brewing. Barley malt and auxiliary materials such as rice are saccharified in the mashing vessel, and the starch is decomposed into maltose to form liquid wort. The mash undergoes solid-liquid separation in the filter tank. The wort enters the fermenter and yeast is added to ferment, and finally beer is produced. The insoluble residue mainly composed of husks is brewer's grains. Unlike white wine grains, there is no yeast and alcohol in brewer's grains, so it is also called spent grains in the industry. Spent grains have high moisture content and are easy to spoil, making them difficult to store for a long time and inconvenient to transport. Currently, most manufacturers in China sell them directly to farmers as feed at low prices, whereas a few manufacturers dry them and use them as raw materials of feed, and some even throw them away directly as waste, wasting resources and seriously damaging the ecological environment, especially in the hot summer.

Spent grains contain a large amount of dietary fiber and protein, accounting for about 50% and 30% respectively. In addition, they also contain phenols, vitamins, mineral elements and other substances, thus they are very nutritious and are high-quality feed resources. However, spent grains has low added value, is difficult for animals to digest, and has low utilization rate if they are sold directly for animal feeding without processing. In recent years, people's demand for meat, eggs and milk has increased dramatically, which has promoted the vigorous development of the breeding industry and the feed industry. The market demand for the feed industry is large, and there is a serious shortage of protein feed. The soybean meal, commonly used as the daily ration, is overly dependent on soybean imports, and the price of feed has risen rapidly. It is urgent to find new feed resources. Spent grains are rich in nutrients and large in amount, which is a good choice. How to turn waste into treasure, make rational use of spent grains, increase added value, and effectively solve environmental pollution and resource waste problems has become a major issue in the beer industry.

At present, there are two ways to use spent grains to make fermented feed: one is to add wet spent grains directly to a dozen or dozens of feed raw materials such as corn flour, bran, etc., and then add some microbial agents or enzyme preparations for fermentation; the other is to dry the wet spent grains first, then add them to a dozen or dozens of feed raw materials, add water to adjust, and then add some microbial agents or enzyme preparations for fermentation. However, the above processing methods have some problems in production process, which are also difficult problems that the feed industry needs to solve.

First, it is regarding the processing method. Since wet spent grains have high moisture content and are rich in nutrients, they will be contaminated by harmful microorganisms in the air if left for a few hours in the hot summer. They are very easy to spoil, and the nutrients are destroyed and toxins are produced. If the distance between the brewery and the feed processing plant is far, or the amount of wet spent grains is large, and the feed production capacity is limited, there will be great risks, which may result in large losses and adverse effects on animals. The first way is to quickly mix and process the wet spent grains, and the second way is to quickly dry the wet spent grains. If the processing is not timely, it will lead to corruption. After drying, the shelf life of the spent grains can be extended, and it is not limited by the time of later mixing and processing, but the cost of drying the spent grains is high, which will increase by 600-1000 yuan per ton or even higher.

Secondly, it is regarding nutrients. At present, when spent grains are used to make feed or fermented feed, they are mostly used as one of a dozen or dozens of raw materials, and the amount added is very small. It is not a feed with spent grains as the main body in the true sense. Due to the low starch content of spent grains (about 3-6%), in the process of preparing fermented feed from spent grains, starch-containing nutrients such as corn flour are often added to supplement the carbon source for animals, but the price of corn flour is relatively high, resulting in higher production costs. If some low-cost substances are added at will, although the cost and moisture content are reduced, the nutritional value of the spent grains themselves is also lowered, and the quality is unstable and difficult to control. The spent grains themselves contain 10-15% cellulose and 10-15% hemicellulose, and this part of the nutrients is not well utilized.

Thirdly, it is regarding fermentation technology. In the process of adding microbial agents and enzyme preparations for solid-state fermentation, the selection of bacterial strains and their addition amount, the selection of enzyme preparations and their addition amount, and conditions of solid-state fermentation process such as fermentation temperature, time, aerobic or anaerobic are all very important and need to be considered systematically and comprehensively, but the research on these parameters are still preliminary at present.

Finally, it is regarding animal experiments. At present, spent grains are mostly used to feed animals directly in an unsophisticated manner, lacking systematic experimental data. In addition, spent grains, especially whole spent grains, are rarely used in animal experiments as fermented feed, which limits the application scope of spent grains as animal feed.

### SUMMARY

In view of at least one shortcoming of the prior art, the present application proposes a fermented feed based on whole spent grains and a preparation method therefor.

The first aspect of the present application provides a method for preparing a fermented feed, comprising: a fresh-keeping treatment step, a drying step, an aerobic fermentation step and an anaerobic fermentation step;
the fresh-keeping treatment step comprises: adding lactic acid bacteria to wet spent grains immediately after taking the wet spent grains;
the aerobic fermentation step comprises: adding mixed microbial community and enzyme preparations to spent grains obtained by the drying step, and performing aerobic fermentation, wherein the mixed microbial community comprises yeast and *Bacillus,* and the enzyme preparations comprise complex cellulases, protease and xylanase; and
the anaerobic fermentation step comprises: adding lactic acid bacteria to the spent grains obtained by the aerobic fermentation step, and performing anaerobic fermentation to obtain the fermented feed.

In some embodiments of the first aspect, the drying step comprises: dehydrating spent grains obtained by the fresh-keeping treatment step until moisture content of the spent grains is 60-70%, and then drying at 55-60°C until the moisture content of the spent grains is 40-50%.

In some embodiments of the first aspect, in the fresh-keeping treatment step, lactic acid bacteria culture solution is added to the wet spent grains, an amount of the lactic acid bacteria culture solution added is 0.5-1% of weight of the wet spent grains, and concentration of the lactic acid bacteria culture solution is not less than 10⁸ cells/mL.

In some embodiments of the first aspect, in the aerobic fermentation step, a mixed microbial community culture solution is added to the spent grains obtained by the drying step, an amount of the mixed microbial community culture solution added accounts for 10-20% of dry weight of the spent grains, the mixed microbial community is composed of the yeast and the *Bacillus* in a ratio of 1:1, and concentration of yeast culture solution and *Bacillus* culture solution is not less than 10⁸ cells/mL.

In some embodiments of the first aspect, in the aerobic fermentation step, an amount of the enzyme preparations added accounts for 1-3% of dry weight of the spent grains, and the enzyme preparations are composed of the complex cellulases, the protease and the xylanase in a ratio of 5:3:2, and enzyme activities of the complex cellulases, the protease and the xylanase are greater than 100,000 U/g.

In some embodiments of the first aspect, the aerobic fermentation step comprises: adding the spent grains to which the mixed microbial community and the enzyme preparations are added into a one-way valve anaerobic fermentation bag, and performing aerobic fermentation at 28-32°C for 1-3 days.

In some embodiments of the first aspect, in the anaerobic fermentation step, adding lactic acid bacteria culture solution to the spent grains obtained by the aerobic fermentation step, an amount of the lactic acid bacteria culture solution added accounts for 10-20% of dry weight of the spent grains, and concentration of the lactic acid bacteria culture solution is not less than 10⁸ cells/mL.

In some embodiments of the first aspect, the anaerobic fermentation step comprises performing anaerobic fermentation at 35-38° C for 4-6 days.

The second aspect of the present application provides a fermented feed based on whole spent grains, the fermented feed prepared by the method for preparing the fermented feed according to any one of the aforementioned technical solutions.

In some embodiments of the second aspect, in the fermented feed: a number of live bacteria is greater than or equal to 10⁸ cells/mL; a weight percentage of crude protein is greater than or equal to 30%; a weight percentage of neutral detergent fiber is greater than or equal to 28% and less than or equal to 40%; a weight percentage of acetic acid is greater than or equal to 0.5% and less than or equal to 2.0%; a weight percentage of lactic acid is greater than or equal to 3% and less than or equal to 5%; and pH is greater than or equal to 3.5 and less than or equal to 4.5.

Compared with the prior art, the beneficial effects of the present application are as follows.
(1) The method for preparing fermented feed provided in at least one embodiment of the present application avoids the risk of contamination by harmful bacteria by specifying the fresh-keeping treatment step, thereby achieving the purpose of preserving wet spent grains and laying the foundation for the subsequent fermentation treatment process; by specifying the aerobic fermentation step and the anaerobic fermentation step, a carbon source is provided for beneficial bacteria and the fermented feed has the function of prebiotics. When the fermented feed is used for animal feeding, the immune ability of the animal can be improved, thereby greatly improving the quality of the fermented feed.
(2) The fermented feed provided in at least one embodiment of the present application is obtained by fermenting whole spent grains, can be used for animal feeding without adding any additional substances, and is functional and beneficial to improving animal immunity; in addition, the feed has good feed palatability and digestibility of nutrients. Based on the nutrition of spent grains and the digestion or milk production mechanism of animals, the nutritional value and effectiveness of whole spent grains fermented feed are confirmed.

### BRIEF DESCRIPTION OF THE DRAWINGS

Fig. 1 is a diagram of a dairy cow eating fermented feed;
Fig. 2 is a diagram of preparing samples and culture medium of ruminants;
Fig. 3 is a chart showing biomimetic digestibility of 0# spent grains and 2# whole spent grains fermented feed in a monogastric animal (duck);
Fig. 4 is a chart showing biomimetic digestibility of 0# spent grains and 2# whole spent grains fermented feed in a monogastric animal (chicken);
Fig. 5 is a chart showing biomimetic digestibility of 0# spent grains and 2# whole spent grains fermented feed in a monogastric animal (pig).

### DETAILED DESCRIPTION OF EMBODIMENTS

The technical solutions in the embodiments will be described clearly and completely below. Apparently, the embodiments to be described are merely some but not all of the embodiments of whole technical solutions in the present application. Based on the overall concept of the present application, all other embodiments obtained by those of ordinary skill in the art shall fall within the protection scope of the present application.

On the one hand, the present application provides a method for preparing a fermented feed, comprising a fresh-keeping treatment step, a drying step, an aerobic fermentation step and an anaerobic fermentation step;
the fresh-keeping treatment step comprises: adding lactic acid bacteria to wet spent grains immediately after taking the wet spent grains;
the aerobic fermentation step comprises: adding mixed microbial community and enzyme preparations to the spent grains obtained in the drying step, and performing aerobic fermentation, wherein the mixed microbial community comprises yeast and *Bacillus,* and the enzyme preparations comprise complex cellulases, protease and xylanase;
the anaerobic fermentation step comprises: adding lactic acid bacteria to the spent grains obtained by the aerobic fermentation step, and performing anaerobic fermentation to obtain the fermented feed.

The above preparation method processes the by-product spent grains of the beer brewing into fermented feed, which can not only greatly increase the added value of the by-product and alleviate the shortage of protein resources in China, but also increase the shelf life of wet spent grains, prevent the spent grains from spoiling and stinking, reduce environmental pollution, increase palatability to animals, and improve the digestibility and utilization rate of nutrients, which is of great significance.

Specifically, in view of the problem that the pretreatment and processing methods of wet spent grains have great risks or high costs, in the present application, lactic acid bacteria are added quickly during or after taking the wet spent grains from the spent grains station, that is, before harmful bacteria are produced in the wet spent grains. The presence of a small amount of lactic acid bacteria can establish a dominant bacterial community, thereby avoiding the risk of contamination by harmful bacteria. Optionally, the lactic acid bacteria are *Pediococcus acidilactici*, which produces acid and bacteriocin, has a wide antibacterial spectrum, inhibits the growth of harmful bacteria, and regulates the gastrointestinal flora.

Furthermore, the wet spent grains are dehydrated and dried by the drying step. Optionally, the drying step comprises: dehydrating the spent grains obtained by the fresh-keeping treatment step until the moisture content of the spent grains is 60-70%, and then drying at 55-60°C until the moisture content of the spent grains is 40-50%. Compared with drying all water in the wet spent grains, the cost is significantly reduced. The technical solution specifically defines that the wet spent grains are first dehydrated to a moisture content of 60-70% by using a dehydrating device, and then dried at 55-60°C until the moisture content of the spent grains is 40-50%. The reason is that this technical means can effectively reduce costs compared with pure baking method.

In order to solve the problem of high cost caused by adding other nutrients, the present application uses spent grains as the single raw material, makes full use of 10-15% cellulose and 10-15% hemicellulose in the spent grains, adds complex cellulases and xylanase, and degrades them into glucose, xylose, arabinose, etc., which not only provides a carbon source, but also has the function of prebiotics, activates beneficial intestinal bacteria and promotes their growth, and improves the body's immune ability. Compared with corn flour, the amount of enzyme preparations added is small, the operation is easy, and the cost is significantly reduced.

In addition, the present application screens the beneficial bacteria and enzyme preparations suitable for fermentation of spent grains, determines the optimal formula of each based on the mixture design, studies the influence of key fermentation processes on the indicators of fermented spent grains, determines the optimal process parameters for spent grains fermentation based on the five-factor experiment, and forms a set of spent grains fermentation technology system covering bacterial agent formula, enzyme preparation formula and key process parameters based on the spent grains raw materials and animal digestion mechanism, which greatly improved the quality of fermented spent grains.

Furthermore, in the technical solution of the present application, a fermentation process of aerobic fermentation followed by anaerobic fermentation is defined. The reason is that yeast and *Bacillus* are aerobic or facultative anaerobes. Under aerobic conditions, yeast and *Bacillus* grow rapidly, produce proteases and flavor substances, and quickly consume free oxygen in the fermentation environment to create a hypoxic or anaerobic environment; then anaerobic lactic acid bacteria are added, and the anaerobic environment created before is suitable for their growth, producing organic acids such as lactic acid and acetic acid, as well as antibacterial substances, lowering the pH value, and inhibiting the growth of pathogenic bacteria.

In some embodiments, in the fresh-keeping treatment step, lactic acid bacteria culture solution is added to the wet spent grains, and the amount of lactic acid bacteria culture solution added is 0.5-1% of the weight of the wet spent grains, and the concentration of the lactic acid bacteria culture solution is not less than 10⁸ cells/mL. The technical solution specifies the amount of lactic acid bacteria added in the fresh-keeping treatment step, because it not only ensures that the content of lactic acid bacteria in the treated spent grains is not less than 10⁵ cells/mL, but also controls the cost of adding lactic acid bacteria, as long as the lactic acid bacteria become dominant bacteria and play a role in temporarily inhibiting harmful bacteria. It is understandable that the proportion of lactic acid bacteria culture solution added can also be 0.6%, 0.7%, 0.8%, 0.9% and any point value within the above range. It is understandable that in the description of the present application, the unit "cells/mL" of the concentration of the bacterial culture solution refers to the number of colony-forming unit, that is, cells/mL=CFU/mL.

In some embodiments, in the aerobic fermentation step, a mixed microbial community culture solution is added to the spent grains obtained by the drying step, and the amount of the mixed microbial community culture solution added accounts for 10-20% of the weight of the dried spent grain (i.e., the dry weight of the spent grains), and the mixed microbial community is composed of the yeast and the *Bacillus* in a ratio of 1:1, and the concentration of yeast culture solution and *Bacillus* culture solution is not less than 10⁸ cells/mL. This technical solution specifies the amount of mixed microbial community added in the aerobic fermentation step to ensure that the number of live bacteria of the mixed microbial community after the spent grains fermentation is not less than 10⁸ cells/mL, so as to produce sufficient fermentation products and control costs. It is understandable that the amount of the mixed microbial community added can also be 12%, 14%, 16%, 18% and any point value within the above range. The present technical solution also specifies that the mixed microbial community is composed of yeast and *Bacillus,* the yeast is optionally *Saccharomyces cerevisiae,* and the *Bacillus* is optionally *Bacillus subtilis,* wherein the yeast produces fragrance, increases palatability, consumes free oxygen, promotes the growth of beneficial bacteria, and synthesizes a variety of enzymes; *Bacillus subtilis* produces spores, has strong stress resistance, consumes free oxygen, promotes the growth of beneficial bacteria, and synthesizes a variety of enzymes. The synthesized enzymes can decompose the macromolecular substances in the spent grains and provide nutrition and prebiotics for the beneficial bacteria.

In some embodiments, in the aerobic fermentation step, the amount of the enzyme preparations added accounts for 1-3% of the dry weight of the spent grains, and the enzyme preparations are composed of the complex cellulases, the protease and the xylanase in a ratio of 5:3:2, and the enzyme activities of the complex cellulases, the protease and the xylanase are greater than 100,000 U/g. This technical solution specifies the amount of enzyme preparations added, because the amount of enzyme preparations added can degrade the cellulose, hemicellulose and protein in the spent grains to generate enough glucose, protein peptides, xylo-oligosaccharides, etc., so as to provide carbon source and nitrogen source and prebiotics for microorganisms, and control costs. It is understandable that the amount of the enzyme preparations added can also be 2%. This technical solution further specifies that the enzyme preparations are composed of complex cellulases, protease and xylanase, and the protease can be optionally acidic protease. The enzyme preparations are added to decompose arabinoxylan to obtain extractable arabinoxylan, which has a certain prebiotic effect. Specifically, complex cellulases is used to degrade macromolecular cellulose to generate glucose, so as to provide an available carbon source for beneficial bacteria; acidic protease is used to degrade macromolecular protein to generate polypeptides and amino acids, so as to provide an available nitrogen source for beneficial bacteria. The acidic protease is selected because a small amount of lactic acid bacteria is added to preserve the spent grains in order to prevent the contamination of harmful microorganisms and cause rancidity during the pretreatment of the spent grains, lactic acid bacteria produce acid and so that the spent grains are acidic. The xylanase is used to degrade arabinoxylan to generate a certain amount of xylo-oligosaccharides and xylose with prebiotic effects. After the above enzymes are added in the aerobic fermentation step, enzyme reactions are carried out in the aerobic fermentation stage (aerobic fermentation at 28-32°C for 1-3 days), and also in the subsequent anaerobic fermentation stage (anaerobic fermentation at 35-38°C for 4-6 days).

In some embodiments, the aerobic fermentation step comprises: adding the spent grains to which the mixed microbial community and the enzyme preparations are added into a one-way valve anaerobic fermentation bag, and performing aerobic fermentation at 28-32°C for 1-3 days. It should be noted that the aerobic fermentation of the present technical solution is to first dehydrate the spent grains and then dry them to obtain spent grains with a moisture content of 40-50% and then perform solid-state fermentation. The present technical solution adopts solid-state fermentation, and compared with liquid fermentation, solid-state fermentation has lower cost, lower mechanization requirements, higher fermentation product concentration, lower pressure and lower energy consumption.

In some embodiments, in the anaerobic fermentation step, a lactic acid bacteria culture solution is added to the spent grains obtained by the aerobic fermentation step, and the amount of the lactic acid bacteria culture solution added accounts for 10-20% of the dry weight of the spent grains, and the concentration of the lactic acid bacteria culture solution is not less than 10⁸ cells/mL. The present technical solution further specifies the amount of lactic acid bacteria added in the anaerobic fermentation step to ensure that the number of live lactic acid bacteria after the spent grains fermentation is not less than 10⁸ cells/mL, so as to produce sufficient fermentation products and control costs. It is understandable that the amount of lactic acid bacteria culture solution added can also be 12%, 14%, 16%, 18% and any point value within the above range.

In some embodiments, the anaerobic fermentation step comprises performing anaerobic fermentation at 35-38° C for 4-6 days. The technical solution further specifies the fermentation conditions of anaerobic fermentation, so that the lactic acid bacteria can grow and reproduce at a suitable temperature, produce enough fermentation products within a suitable time, shorten the fermentation cycle, and reduce costs.

On the other hand, the present application provides a fermented feed based on whole spent grains, which is prepared by the preparation method of the fermented feed described in any of the above technical solutions. The fermented feed is obtained by fermenting whole spent grains, can be used for animal feeding without adding any additional substances, and is functional and beneficial to improve animal immunity. Through systematic multi -dimensional animal experiments on the fermented feed based on whole spent grains, including feeding tests on dairy cows, vitro biomimetic tests on ruminants and monogastric animals, and rumen digestion tests on fistulated cows, the results show that the fermented feed based on whole spent grains of the present application significantly increases the feed intake of animals, improves the palatability of feed and the digestibility of nutrients, and the nutritional value and effectiveness of the whole spent grains fermented feed are confirmed based on the nutrition of spent grains and the digestion or milk production mechanism of animals.

Furthermore, the fermented feed prepared by the above preparation method has an obvious and pleasant fermentation sour aroma, no musty smell or other peculiar smell, is softer and looser than the spent grains before fermentation, the husk becomes softer, and the palatability is significantly improved. Specifically: a number of live bacteria is greater than or equal to 10⁸ cells/mL; a weight percentage of crude protein is greater than or equal to 30%; a weight percentage of neutral detergent fiber is greater than or equal to 28% and less than or equal to 40%; a weight percentage of acetic acid is greater than or equal to 0.5% and less than or equal to 2.0%; a weight percentage of lactic acid is greater than or equal to 3% and less than or equal to 5%; and pH is greater than or equal to 3.5 and less than or equal to 4.5.

In order to more clearly and in detail introduce the fermented feed based on whole spent grains and the preparation method thereof provided in the embodiments of the present application, specific embodiments will be described below.

### Embodiment 1

### 1. Microorganisms

The lactic acid bacteria, *Saccharomyces cerevisiae* and *Bacillus subtilis,* which had excellent antibacterial and protease production abilities, screened out in the laboratory, were activated stepwise and cultured scale-up. The microbial culture solutions were cultured scale-up according to the weight of the wet spent grains. 5 kg of wet spent grains were taken from the spent grains station of the brewery. Due to the total amount of microbial culture solutions added was 10% of the weight of the wet spent grains and each of the three microbial culture solutions accounts for about 1/3, about 200 mL of the three microbial culture solutions needed to be cultured scale-up.
(1) First activation. Took out the glycerol cryo vials of the bacteria from the -80°C freezer. After the cryovials were thawed, took 100 µL bacteria from the glycerol cryovials respectively into 5 mL liquid culture media in test tubes for activation (MRS liquid culture medium for *Pediococcus acidilactici*, cultured in a 37°C incubator for 24 h; YPD liquid culture medium for *Saccharomyces cerevisiae,* cultured in a 30°C incubator for 24 h; LB liquid culture medium for *Bacillus subtilis,* cultured in a 37°C shaker with shaking for 24 h).
(2) Second activation. Took 200 µL from each of the first activation liquid test tubes and transfered them respectively to a 20 mL liquid culture medium in Erlenmeyer flask for secondary activation (MRS liquid culture medium for *Pediococcus acidilactici*, cultured in a 37°C incubator for 24 h; YPD liquid culture medium for *Saccharomyces cerevisiae,* cultured in a 30°C incubator for 24 h; LB liquid culture medium for *Bacillus subtilis,* cultured in a 37°C shaker with shaking for 24 h).
(3) Cultivation. Took 10 mL from each of the secondary activation liquid test tubes and transfered them respectively to a 200 mL liquid culture medium in Erlenmeyer flask for cultivation (MRS liquid culture medium for *Pediococcus acidilactici*, cultured in a 37°C incubator for 24-48 h; YPD liquid culture medium for *Saccharomyces cerevisiae,* cultured in a 30°C incubator for 24-48 h; LB liquid culture medium for *Bacillus subtilis,* cultured in a 37°C shaker with shaking for 24-48 h).
(4) Determination of live bacteria counts in cultured bacterial suspension.

There are three common methods for determining live bacteria counts:
1) Turbidity method. Take 200 µL of the mixed microbial community solution and place it in an ELISA plate, using the corresponding blank liquid culture medium as a control. Measure the optical density at 600 nm, ensuring the optical density is greater than 2.0 to meet the requirement.
2) Dilution plate count method. Take 100 L from the bacterial suspension, add 900 µL sterile water, shake and mix, and put it into tube 1#, with a dilution factor of 10; take 100 µL from tube 1#, add 900 µL sterile water, shake and mix, and put it into tube 2#, with a dilution factor of 100; dilute to 10⁶ in sequence, with a total of 6 dilution gradients. Prepare MRS, YPD, and LB solid plate culture media, and select three dilutions of 10⁴, 10⁵ and 10 ⁶ to spread on the plates. Culture the MRS and LB plates at 37°C for 48 h, and place the YPD plate at 30°C for 48 h. Record the dilution factors and the corresponding numbers of colonies on the plates. Calculate the average number of colonies on the two parallel plates, and then multiply the average number by the corresponding dilution factor as the total number of colonies per mL. The results of the turbidity method and the dilution plate method are highly correlated. The turbidity method is simple and fast, but relatively rough; the dilution plate method is accurate, but the method is cumbersome and lagging. The two methods complement each other. Generally, the turbidity method is used to determine the number of live bacteria in the culture suspension, and the dilution plate method is used to determine the number of live bacteria in the whole spent grains fermented feed.
3) Automatic counting instrument method. Currently, there is an automatic counting instrument for yeast, and the method of determining the number of yeast cells is relatively mature and has been widely used. However, bacteria are small in size and there is no mature instrument yet.

OD (Optical Density) values of the three bacterial suspensions at 600 nm were measured by the turbidity method, as shown in Table 1.

**Table 1 Numbers of live bacteria in three culture suspensions (turbidity method)**

| Serial number | M 5-3 | Y 10-1 | L 3-2 |
|---|---|---|---|
| Bacteria name | *Pediococcus acidilactici* | *Saccharomyces cerevisiae* | *Bacillus subtilis* |
| Liquid culture medium used | MRS | YPD | LB |
| OD value at 600 nm | 2.734 | 2.438 | 2.366 |

### 2. Pretreatment of spent grains

(1) Took 5 kg of wet spent grains from the brewery's spent grains station, immediately add 25 mL of the culture solution of *Pediococcus acidilactici*, and stirred evenly.
(2) After the wet spent grains were transported to the laboratory, the moisture content of the spent grains was measured: 5 g was accurately weighed in a weighing bottle, and the total weight of the empty bottle and the whole weight with spent grains were weighed. The spent grains were dried in an oven at 105°C for 3.5 h, and the total weight after drying was measured. The moisture content of the spent grains was calculated to be 81%, and the dry weight was 19%.
(3) The wet spent grains were dehydrated using a dehydrator, and the actual moisture content of the dehydrated spent grains was measured according to the above method, showing that the moisture content was reduced from 81% to 65%. The dehydrated spent grains were placed in an oven and dried at 55°C for 4 h, and the actual moisture content of the dried spent grains was measured according to the above method, showing that the moisture content was reduced from 65% to 45%. After deducting the loss and intermediate sampling, 1.5 kg of baked spent grains were obtained.

### 3. Preparation of whole spent grains fermented feed

(1) The dry weight of the dried spent grains was calculated to be 825 g. 10% (82 mL) of culture solutions of *Saccharomyces cerevisiae* and *Bacillus subtilis* were added to the dried spent grains in a ratio of 1:1. The actual amount of addition was calculated based on the OD value of the culture solutions: 40 mL of *Saccharomyces cerevisiae* and 42 mL of *Bacillus subtilis;* stirred them evenly.
(2) 2% enzyme preparations were added to the spent grains with bacteria added, the total dry weight of the enzyme preparations were 16.5 g, including complex cellulases, acid protease, and xylanase, in a ratio of 5:3:2. The actual addition amounts of the three enzymes were 8.25 g, 4.95 g, and 3.3 g, respectively.
(3) The spent grains after adding bacteria and enzyme preparations were placed into a one-way valve anaerobic fermentation bag, stirred evenly, did not seal the bag, and fermented aerobically at 30°C for 2 days.
(4) 10% (based on dry weight) lactic acid bacteria with a total amount of 82 mL were added to the spent grains, stirred evenly, sealed the fermentation bag, and fermented anaerobically at 37°C for 5 days to obtain whole spent grains fermented feed with spent grains as the only raw material.

### Embodiment 2

The prepared whole spent grains fermented feed was compared with the initial unfermented spent grains and commercially available fermented feeds, as shown in Table 2. All indicators are based on dry weight.

**Table 2 Comparison of whole spent grains fermented feed, unfermented spent grains, and commercially available fermented feeds**

| Serial number | Name | Moisture % | pH | Acetic acid % | Lactic acid % | Crude Protein % | Neutral detergent fiber % | Acid detergent fiber % |
|---|---|---|---|---|---|---|---|---|
| 1 | Whole spent grains fermented feed | 45 | 3.8 | 1.6 | 4.2 | 33.4 | 35.7 | 28.3 |
| 2 | Unfermented spent grains | 81 | 6.1 | 0.03 | 0.05 | 28.7 | 48.5 | 37.2 |
| 3 | Commercially available fermented feed 1 | 63 | 4.8 | 1.1 | 2.6 | 24.5 | 43.6 | 34.9 |
| 4 | Commercially available fermented feed 2 | 37 | 4.1 | 0.45 | 3.4 | 15.2 | 41.8 | 33.5 |

Lactic acid bacteria produce fatty acids, causing a decrease in pH, which can effectively inhibit harmful bacteria. Short-chain volatile fatty acids are involved in the regulation of feeding, among which acetic acid is a precursor for milk fat synthesis. Protein is the main nutrient required by animals. Neutral detergent fiber and acid detergent fiber are used to evaluate fiber and nutrition of daily rations, and high contents thereof are not conducive to animal digestion and affect palatability.

First, the differences before and after the fermentation of the spent grains were compared. After fermentation, all indicators were significantly improved. Short-chain fatty acids (acetic acid, lactic acid) increased, crude protein content increased, and neutral and acidic detergent fiber decreased significantly. After fermentation, there was a pleasant sour aroma.

Compared with two commercially available fermented feeds, the whole spent grains fermented feed had higher acetic acid, lactic acid and crude protein contents, and lower neutral and acid detergent fiber contents.

The whole spent grains fermented feed of the present application is superior to unfermented spent grains and the two commercially available fermented feeds in all indicators.

### Embodiment 3

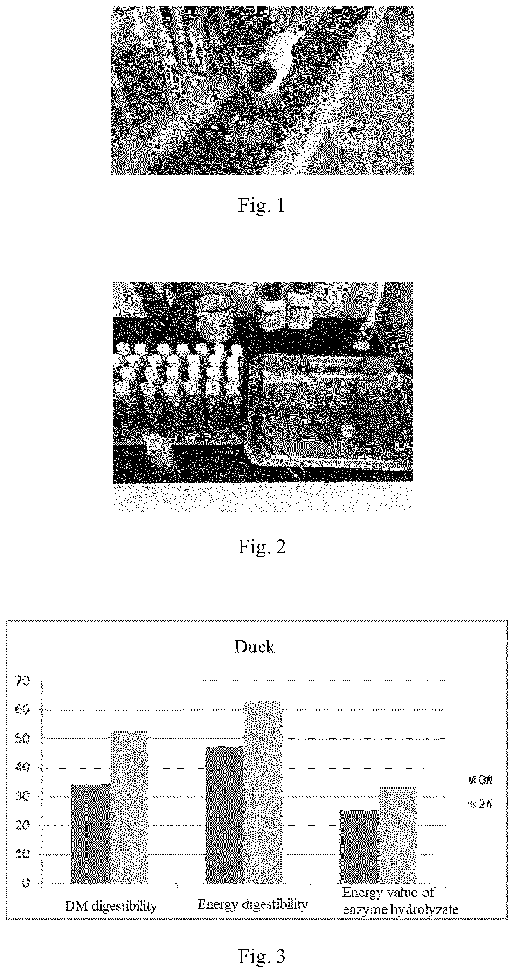

The whole spent grains fermented feed prepared in the present application, unfermented spent grains, and three commercially available fermented feeds were used to conduct feeding experiments on dairy cows, as shown in Fig. 1, to evaluate the application effect of the fermented feed based on whole spent grains on animals and investigate the palatability. The five samples were respectively unfermented spent grains 1#, fermented feed based on whole spent grains 2#, and commercially available fermented feeds 3-5#, each weighing about 1 kg.

In a dairy farm, multiple cows were selected and fed with the five samples respectively to observe the cows' preference for the five samples, and a single cow was fed multiple times to observe the repeatability. The remaining amount of samples was also be used for judgment.

The results showed: The feeding priority of the five samples were 2#>3#>1#>4#>5#. The whole spent grains fermented feed of the present application had a pleasant sour aroma and was eaten the fastest by cows, indicating that cows like it the most and it has the best palatability. The sample 3# was fermented soybean meal, the soybean meal was one of the daily rations for dairy cows and after fermentation, the aroma was increased and the palatability was increased. Unfermented spent grains were also feed for dairy cows, but they had a sour smell and high moisture content, so cows didn't like them. The sample 4# was a fermented feed containing spent grains, but the proportion of spent grains was very small, and it was mainly silage. The 5# fermented feed did not contain spent grains, but contained bran, corn stalks, etc., and has a slight ammonia smell.

It shows that raw materials, appropriate bacterial-enzyme synergy, and appropriate conditions of fermentation process of feed are all key factors in preparing fermented feed.

### Embodiment 4

The whole spent grains fermented feed prepared in the present application, unfermented spent grains and two commercially available fermented feeds were subjected to animal biomimetic evaluation tests in vitro.

### 1. Evaluation method of in vitro digestibility of ruminants

(1) Each feed was dried in an oven at 65°C for 12 h until constant weight was reached, and placed in a sealable bag for later use.
(2) Each feed was passed through a 40-mesh sieve by a plant crusher.
(3) The dry matter (DM) content and crude protein (CP) content of the raw materials were determined, referring to the national standard.
(4) Fermentation culture solution was prepared according to Menke's method.
(5) Cows were slaughtered before morning feeding and the rumen fluid was collected. The rumen fluid was filtered into a household thermos filled with CO₂ by using four layers of medical gauze
(6) The pH of the fermentation culture solution was adjusted to 7.0-7.3, the fermentation culture solution and rumen fluid were mixed in a ratio of 2:1, poured into a 2 L beaker, placed in a 39°C water bath, and stirred at a constant speed to prepare the artificial culture solution.
(7) 1.0000 g ± 0.0010 g of sample was weighted into a disposable sample bottle, 60 mL of artificial culture solution was added, sealed the bottle, placed in a 39°C water bath shaker at 180 rpm, and reacted for 24 h. See Fig. 2.
(8) After the reaction was completed, the pH of the reaction solution was measured, and the precipitate was collected and its DM and CP contents were measured.
(9) Calculation Nutrient digestibility = (nutrient content before reaction - nutrient content after reaction) ÷ nutrient content before reaction × 100%

The in vitro digestibility in the rumen of ruminants of the four fermented feeds is shown in Table 3.

**Table 3 In vitro digestibility of different fermented feeds in the rumen of ruminants**

| Group | Unfermented spent grains | Commercially available fermented feed1 | Whole spent grains fermented feed | Commercially available fermented feed 2 |
|---|---|---|---|---|
| DM digestibilit y% | 24.91 | 55.75 | 78.96 | 56.82 |
| NDF digestibilit y% | 9.47 | 23.12 | 27.49 | 13.53 |

High digestibility, increased feed intake and good absorption of animals will synthesize more nutrients and improve production performance. Compared with unfermented spent grains, the digestibility of dry matter (DM) and neutral detergent fiber (NDF) of the whole spent grains fermented feed is significantly increased, indicating that fermentation improves the rumen digestibility of ruminants. After spent grains are fermented, macromolecules are degraded and easier to be digested by dairy cows. It is also better than two commercially available fermented feeds.

Commercially available fermented feed 1 did not contain *Bacillus subtilis,* may have resulted in less cellulase secretion, which reduced the degradation of cellulose and affected the digestibility.

The digestibility of commercially available fermented feed 2 is relatively low, and its fiber content is relatively high, which reduces its digestibility. It is speculated that high-fiber raw material was added during the preparation process, and it is not conducive to the digestion and utilization of animals.

### 2. Evaluation of biomimetic in vitro digestibility of monogastric animals (ducks, chickens, pigs)

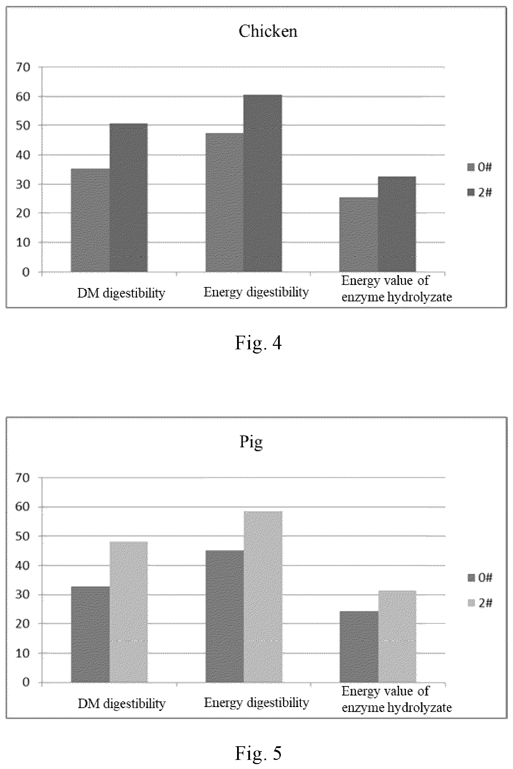

The SDS-III monogastric animal biomimetic digestion system was used to automatically simulate the gastrointestinal digestion process of pigs and poultry, and the differences in digestion indicators between unfermented spent grains 0# and fermented feed based on whole spent grains 2# were analyzed. The results are shown in Figs. 3-5.

The SDS-III monogastric animal biomimetic digestion system consists of a simulated digester and a control system. The inside of the dialysis bag in the simulated digester is regarded as the internal environment (digestive environment) of the stomach, small intestine, and large intestine, and the outside of the dialysis bag is regarded as the capillary body fluid environment (absorption environment). The configuration software technology is used to make the buffer solution automatically enter input-emptying-switching system, digestive fluid automatic secretion system, hydrolysis product automatic cleaning system, constant temperature control system, mixing intensity automatic control system, electronic component signal detection system and other modules in the simulated digester through computer program control to achieve full automatic simulation of the gastrointestinal digestion process of pigs and poultry.

The results showed that compared with unfermented spent grains, the dry matter (DM) digestibility, energy digestibility and energy value of enzyme hydrolyzate of the whole spent grains fermented feed were significantly increased. This shows that the whole spent grains fermented feed is also suitable for monogastric animals such as ducks, chickens and pigs. The biomimetic digestibility of the whole spent grains fermented feed for the three monogastric animals from high to low are: ducks>chickens>pigs.

### Embodiment 5

### (1) Evaluation of the rumen degradation of whole spent grains fermented feed and unfermented spent grains in fistulated cows

Three healthy Holstein cows with permanent fistulas were selected, and samples (whole spent grains fermented feed and unfermented malt grain) were weighed into special nylon bags. Three parallels were set for each cow at each time point, and the samples were put into the rumen before feeding in the morning and were taken out after 2 h, 4 h, 8 h, 12 h, 24 h, 36 h, and 48 h of incubation. They were placed in a 55°C oven for 48 h to reach constant weight, and the rumen degradation rates of DM, NDF, and CP of the samples at different time points were determined. The rapidly degradable part a, the slowly degradable part b, and the rumen effective degradation rate ED were calculated.

Rumen degradation rate and degradation parameters of dry matter in unfermented spent grains and whole spent grains fermented feed are shown in Table 4.

**Table 4 Rumen degradation rate and degradation parameters of dry matter in unfermented spent grains and whole spent grains fermented feed**

| Items | Unfermented spent grains | Whole spent grains fermented feed | Standard error | P-value |
|---|---|---|---|---|
| Rumen degradation rate /% | | | | |
| 2h | 16.5 | 44.2 | 0.869 | <0.0001 |
| 4h | 18.2 | 48.7 | 1.29 | <0.0001 |
| 8h | 30.9 | 49.5 | 2.70 | 0.0083 |
| 16h | 40.5 | 55.6 | 3.90 | 0.05 |
| 24h | 42.3 | 58.7 | 1.63 | 0.0020 |
| 36h | 59.7 | 65.8 | 1.10 | 0.0175 |
| 48h | 62.9 | 69.9 | 1.75 | 0.7846 |
| Rapidly degradable part a/% | 11.2 | 38.6 | 0.770 | <0.0001 |
| Slowly degradable part b/% | 78.1 | 35.3 | 6.53 | 0.0098 |
| Degradation rate of the slowly degradable part (%/h) | 2.81 | 5.14 | 1.11 | 0.2107 |
| Rumen effective degradation rate /% | 38.5 | 55.0 | 1.10 | 0.0004 |

Rumen degradation rate of dry matter (DM) can reflect the ease of digestion of feed in the rumen, and is also an important factor affecting the DM intake of dairy cows. The results showed that the rumen degradation rate and effective degradation rate (ED) of DM in the whole spent grains fermented feed increased significantly over time and were significantly higher than those of unfermented spent grains. The DM was mainly degraded within 36 hours, and the degradation rate at 48 hours was about 70%.

Rumen degradation rate and degradation parameters of crude protein are shown in Table 5. The degradation of crude protein (CP) in the rumen is mainly determined by its retention time in the rumen and the difficulty of degradation. In addition, the degradation of CP in the rumen is also directly related to its internal structure composition, non-protein nitrogen (NPN) and true protein content, the physical and chemical properties of true protein, the presence of cell wall inert barriers and anti-nutritional factors. The results of this experiment showed that the rumen degradation rate, value a and ED value of CP in the whole spent grains fermented feed increased significantly. It may be that the reduction in DM content relatively increased the concentration of proteolytic enzymes in the rumen, resulting in an increase in CP degradation rate. It may also be because the fermentation process can increase the content of NPN and soluble protein, increase the value a of CP in the feed, and thus increase the degradation rate of CP.

**Table 5 Rumen degradation rate and degradation parameters of crude protein in unfermented spent grains and whole spent grains fermented feed**

| Items | Unfermented spent grains | Whole spent grains fermented feed | Standard error | P-value |
|---|---|---|---|---|
| Rumen degradation rate /% | | | | |
| 2h | 10.6 | 56.0 | 1.23 | <0.0001 |
| 4h | 10.7 | 59.4 | 2.39 | 0.0001 |
| 8h | 22.3 | 61.0 | 4.27 | 0.0036 |
| 16h | 22.8 | 67.6 | 3.84 | 0.0012 |
| 24h | 41.5 | 77.2 | 2.90 | 0.001 |
| 36h | 74.7 | 80.6 | 0.533 | 0.0014 |
| 48h | 87.1 | 89.7 | 4.20 | 0.684 |
| Rapidly degradable part a /% | 1.12 | 45.3 | 2.03 | 0.0001 |
| Slowly degradable part b /% | 98.8 | 45.4 | 2.03 | <0.0001 |
| Degradation rate of the slowly degradable part (% /h) | 2.93 | 5.25 | 0.507 | 0.0317 |
| Rumen effective degradation rate /% | 37.5 | 68.3 | 1.70 | 0.0002 |

Rumen degradation rate and degradation parameters of neutral detergent fiber in unfermented spent grains and whole spent grains fermented feed are shown in Table 6. The rumen degradation rate of NDF is an important indicator of the nutritional value of feed, and the composition of NDF will affect the rumen degradation rate of NDF. After fermentation, the rumen degradation rate of NDF of the whole spent grains fermented feed at each time point increased significantly. It may be that the NDF and DM content of spent grains decreased after microbial fermentation, resulting in an increase in the concentration of cellulase and hemicellulase that decompose NDF, thereby increasing the rumen degradation rate and ED value of NDF.

**Table 6 Rumen degradation rate and degradation parameters of neutral detergent fiber in unfermented spent grains and whole spent grains fermented feed**

| Items | Unfermented spent grains | Whole spent grains fermented feed | Standard error | P-value |
|---|---|---|---|---|
| Rumen degradation rate /% | | | | |
| 2h | 13.6 | 37.0 | 0.58 | <0.0001 |
| 4h | 16.9 | 37.7 | 1.43 | 0.0006 |
| 8h | 25.0 | 38.5 | 1.88 | 0.0071 |
| 16h | 29.2 | 43.9 | 2.46 | 0.0135 |
| 24h | 41.0 | 42.9 | 1.82 | 0.5103 |
| 36h | 49.8 | 56.2 | 1.52 | 0.0414 |
| 48h | 57.3 | 62.1 | 1.52 | 0.0868 |
| Rapidly degradable part a/% | 7.27 | 32.1 | 1.28 | 0.0002 |
| Slowly degradable part b/% | 65.2 | 27.2 | 4.41 | 0.0037 |
| Degradation rate c of the slowly degradable part (%/h) | 3.24 | 3.97 | 0.651 | 0.4742 |
| Rumen effective degradation rate ED/% | 31.9 | 43.7 | 0.625 | 0.0002 |

### (2) Evaluation of the rumen degradation of conventional daily ration and whole spent grains fermented feed in fistulated cows

4.35 kg of whole spent grains fermented feed was added to the daily ration, accounting for 14.41% of the dry matter of the daily ration, replacing 0.52 kg of the daily ration (including soybean meal and other fermented feed, etc.). The replacement scheme was calculated by the CPM dairy cow formula software, and the protein content, dry matter content, and fiber content remained basically unchanged before and after the replacement. The conventional daily ration and the daily ration with whole spent grains fermented feed added were used for fistulated cow experiments to analyze the rumen degradation rate and effective degradation rate of protein and fiber, as shown in Tables 7 and 8. The results showed that compared with the conventional daily ration, whole spent grains fermented feed increased the rumen degradation rate of crude protein, and the rumen degradation rate of neutral detergent fiber remained basically unchanged.

**Table 7 Rumen degradation rate and degradation parameters of crude protein**

| Items | Conventional daily ration | Daily ration with whole spent grains fermented feed added | Standard error | P-value |
|---|---|---|---|---|
| Rumen degradation rate /% | | | | |
| 2h | 47.2 | 49.5 | 2.20 | 0.5002 |
| 4h | 56.7 | 64.0 | 2.04 | 0.0641 |
| 8h | 57.2 | 60.3 | 1.48 | 0.2126 |
| 16h | 67.3 | 70.5 | 2.10 | 0.3361 |
| 24h | 75.1 | 75.8 | 1.73 | 0.8032 |
| 36h | 85.6 | 85.5 | 1.81 | 0.9690 |
| 48h | 92.4 | 96.5 | 0.388 | 0.0018 |
| Rapidly degradable part a/% | 48.0 | 53.3 | 1.49 | 0.0657 |
| Slowly degradable part b/% | 52.0a | 45.8b | 1.33 | 0.0302 |
| Degradation rate of the slowly degradable part (%/h) | 5.67 | 5.26 | 0.453 | 0.5543 |
| Rumen effective degradation rate /% | 75.6 | 76.7 | 0.869 | 0.4334 |

**Table 8 Rumen degradation rate and degradation parameters of neutral detergent fiber**

| Items | Conventional daily ration | Daily ration with whole spent grains fermented feed added | Standard error | P-value |
|---|---|---|---|---|
| Rumen degradation rate /% | | | | |
| 2h | 36.1 | 40.1 | 1.43 | 0.1175 |
| 4h | 38.1 | 40.2 | 0.673 | 0.0939 |
| 8h | 44.5 | 41.4 | 1.56 | 0.2364 |
| 16h | 39.1 | 40.7 | 1.59 | 0.5208 |
| 24h | 45.9 | 48.1 | 1.01 | 0.2082 |
| 36h | 52.4 | 53.8 | 2.24 | 0.6884 |
| 48h | 55.0 | 62.0 | 0.619 | 0.0013 |
| Rapidly degradable part a/% | 36.6 | 36.9 | 1.11 | 0.8402 |
| Slowly degradable part b/% | 63.5 | 63.1 | 1.11 | 0.8402 |
| Degradation rate of the slowly degradable part (%/h) | 0.89 | 0.86 | 0.059 | 0.7843 |
| Rumen effective degradation rate /% | 46.1 | 46.2 | 0.861 | 0.9696 |

## Claims

1. A method for preparing a fermented feed, comprising: a fresh-keeping treatment step, a drying step, an aerobic fermentation step and an anaerobic fermentation step;
the fresh-keeping treatment step comprises: adding lactic acid bacteria to wet spent grains immediately after taking the wet spent grains;
the aerobic fermentation step comprises: adding mixed microbial community and enzyme preparations to spent grains obtained by the drying step, and performing aerobic fermentation, wherein the mixed microbial community comprises yeast and *Bacillus,* and the enzyme preparations comprise complex cellulases, protease and xylanase; and
the anaerobic fermentation step comprises: adding lactic acid bacteria to the spent grains obtained by the aerobic fermentation step, and performing anaerobic fermentation to obtain the fermented feed.

2. The method for preparing the fermented feed according to claim 1, **characterized in that**, the drying step comprises: dehydrating spent grains obtained by the fresh-keeping treatment step until moisture content of the spent grains is 60-70%, and then drying at 55-60°C until the moisture content of the spent grains is 40-50%.

3. The method for preparing the fermented feed according to claim 1, **characterized in that**, in the fresh-keeping treatment step, lactic acid bacteria culture solution is added to the wet spent grains, an amount of the lactic acid bacteria culture solution added is 0.5-1% of weight of the wet spent grains, and concentration of the lactic acid bacteria culture solution is not less than 10⁸ cells/mL.

4. The method for preparing the fermented feed according to claim 1, **characterized in that**, in the aerobic fermentation step, a mixed microbial community culture solution is added to the spent grains obtained by the drying step, an amount of the mixed microbial community culture solution added accounts for 10-20% of dry weight of the spent grains, the mixed microbial community is composed of the yeast and the *Bacillus* in a ratio of 1:1, and concentration of yeast culture solution and *Bacillus* culture solution is not less than 10⁸ cells/mL.

5. The method for preparing the fermented feed according to claim 1, **characterized in that**, in the aerobic fermentation step, an amount of the enzyme preparations added accounts for 1-3% of dry weight of the spent grains, and the enzyme preparations are composed of the complex cellulases, the protease and the xylanase in a ratio of 5:3:2, and enzyme activities of the complex cellulases, the protease and the xylanase are greater than 100,000 U/g.

6. The method for preparing the fermented feed according to claim 1, **characterized in that**, the aerobic fermentation step comprises: adding the spent grains to which the mixed microbial community and the enzyme preparations are added into a one-way valve anaerobic fermentation bag, and performing aerobic fermentation at 28-32°C for 1-3 days.

7. The method for preparing the fermented feed according to claim 1, **characterized in that**, in the anaerobic fermentation step, adding lactic acid bacteria culture solution to the spent grains obtained by the aerobic fermentation step, an amount of the lactic acid bacteria culture solution added accounts for 10-20% of dry weight of the spent grains, and concentration of the lactic acid bacteria culture solution is not less than 10⁸ cells/mL.

8. The method for preparing the fermented feed according to claim 1, **characterized in that**, the anaerobic fermentation step comprises performing anaerobic fermentation at 35-38° C for 4-6 days.

9. A fermented feed based on whole spent grains, **characterized in that**, the fermented feed prepared by the method for preparing the fermented feed according to any one of claims 1-8.

10. The fermented feed based on whole spent grains according to claim 9, **characterized in that**, in the fermented feed: a number of live bacteria is greater than or equal to 10⁸ cells/mL; a weight percentage of crude protein is greater than or equal to 30%; a weight percentage of neutral detergent fiber is greater than or equal to 28% and less than or equal to 40%; a weight percentage of acetic acid is greater than or equal to 0.5% and less than or equal to 2.0%; a weight percentage of lactic acid is greater than or equal to 3% and less than or equal to 5%; and pH is greater than or equal to 3.5 and less than or equal to 4.5.
